# EUROPEAN PATENT SPECIFICATION

(11) **EP 1 618 871 B1**
(45) Date of publication and mention of the grant of the patent: **28.05.2008**
(21) Application number: 04291691.6
(22) Date of filing: 02.07.2004
(51) Int. Cl.: A61K 8/896, A61Q 17/02, A61Q 19/04

(54) **Sunscreen composition comprising a silicon modified hydrolysed protein**
Silikon-modifiziertes Protein enthaltende Sonnenschutzzubereitung
Composition solaire comprenant une silicone modifiée avec une protéine hydrolysée

(43) Date of publication of application: 25.01.2006
(73) Proprietor: Johnson & Johnson Consumer France SAS, 92787 Issy les Moulineaux Cedex 9 (FR)
(72) Inventor: Brillouet, Anne Sophie, 27400 Louviers (FR); Tischenbach, Isabelle, 27400 Louviers (FR)
(74) Representative: Weber-Bruls, Dorothée

(56) References cited:
- FR-A- 2 787 798
- US-A- 4 559 225
- US-A- 5 447 715
- US-A- 5 753 214
- US-B1- 6 268 454
- PATENT ABSTRACTS OF JAPAN vol. 2000, no. 08, 6 October 2000 (2000-10-06) & JP 2000 136115 A (SEIWA KASEI:KK), 16 May 2000 (2000-05-16)

## Description

### FIELD OF THE INVENTION

The present invention relates to a sunscreen composition having excellent rubbing resistance and global remanence after a single application.

### BACKGROUND OF THE INVENTION

The prolonged exposure to ultra-violet (UV) radiation, such as from the sun, can lead to the formation of light dermatoses and erythemas, as well as increase the risk of skin cancers, such as melanoma. UV radiation can also accelerate skin aging, such as loss of skin elasticity and wrinkling. Radiation with wavelengths in the UV-A range (from about 320 to 400 nm) and the UV-B range (from about 280 to about 320 nm) can cause such skin damage, and, thus, sunscreen products should preferably comprise both UV-A and UV-B sun filters.

Commercially available sunscreen products with low to high sun protection factor (SPF) are available. However, they typically require reapplication after a few hours, especially after exposure to water, perspiration, sand, and towel rubbing.

The present invention relates to a sunscreen capable of providing excellent rubbing resistance and long lasting remanence on the skin, with low to high SPF and acceptable consistency, skin feel and stability. In one embodiment, the sunscreen product comprises a silicon modified hydrolyzed protein.

US Patent No. 5,753,214 discloses the use of silylated peptides for use in cosmetics, particularly hair care products such as shampoo. The patent generally discloses that the silylated peptides may also be used in a variety of skin care applications including anti-suntan and anti-sunburn preparations. However, no further information is provided, particularly as to remanence, water resistance, sand resistance, and combination with other ingredients in suncare products such as film-forming agents.

Document US 5,753,214 discloses a specific silylated peptide which is soluble in water and has excellent pH stability and storage stability in water, which, moreover, has both excellent properties as derived from silicone oils and excellent properties as derived from polypeptides when incorporated into hair cosmetics, and which imparts gloss and moisture to the hair, and improves its combability, and prevents hair from splitting.

This specific silylated peptide is reported to be applicable to various cosmetics such as shampoos, hair rinses, split hair coatings, first and second solutions for permanent waving treatment, hair creams, hair conditioners, set lotions, hair colours or hair dyes, hair treatment rinses, liquid hair dressings, hair packs, hair tonics and other hair cosmetics, toilet waters, after-shaving lotions, shaving foams, vanishing creams, cleansing creams, emollient creams, moisture creams, hand creams and rolling massage creams, depilatories, face packs, emulsions, cleaning preparations, body shampoos, various soaps, make-ups, anti-suntan and anti-sunburn preparations.

### SUMMARY OF THE INVENTION

The invention relates to a sunscreen product according to the features of claim 1. This product has rubbing resistance for at least 5 hours after a single application to mammalian skin or hair and an SPF of at least 2.

The invention also relates to a composition comprising a) from 0.1 to 10 weight percent of at least one silicon modified hydrolyzed protein; b) less than 3 weight percent of at least one film-forming agent; and c) from 1 to 30 weight percent of a sunscreen containing at least one UV-A absorber, UV-B absorber, infrared protector, or combination thereof.

The invention further relates to a sunscreen product comprising a) at least one silicon modified hydrolyzed protein; and b) less than 3 weight percent of a film-forming agent; wherein said product has a rubbing resistance for at least 5 hours after a single application to mammalian skin and an SPF of at least 2.

Finally, the invention also relates to a sunscreen product having a global remanence of at least 8 hours.

Other features and advantages of the present invention will be apparent from the detailed description of the invention and from the claims.

### DETAILED DESCRIPTION OF THE INVENTION

Unless defined otherwise, all technical and scientific terms used herein have the same meaning as commonly understood by one of ordinary skill in the art to which the invention belongs. Unless otherwise indicated, a percentage refers to a percentage by weight (i.e., %(W/W)).

As used herein, "cosmetically-acceptable" means that the ingredients the term describes are suitable for use in contact with tissues (e.g., the skin or hair) without undue toxicity, incompatibility, instability, irritation, allergic response, and the like.

As used herein, "safe and effective amount" means an amount sufficient to achieve a cosmetic or therapeutic effect, but low enough to avoid serious side effects. The safe and effective amount of the product or composition will vary with the area being treated, the age and skin type of the end user, the duration and nature
of the treatment, the specific ingredient or composition employed, the particular cosmetically-acceptable carrier utilized, and like factors.

The present sunscreen product advantageously has a rubbing resistance for at least 5 hours after a single application to mammalian skin or hair. Rubbing resistance is tendency not to be removed from skin or hair by contact with sand, clothes, towels, or other fabrics. Preferably, the sunscreen product has a rubbing resistance for at least 6, more preferably 10, hours after a single application to mammalian skin or hair.

In one embodiment, the sunscreen product also has a global remanence for at least 8 hours, preferably at least 10 hours, after a single application to mammalian skin or hair. Global remanence is defined as maintenance of at least 50 % of the initial level of one or more of the following: UV-A protection, UV-B protection, water resistance, and rubbing resistance. Current commercially available sunscreen products have a remanence, primarily water remanence, only up to 2 hours. Typical recommendations for commercially available products are to reapply every 2 hours.

One measurement of rubbing resistance is sand resistance. The sunscreen product resistance of at least 65%. Preferably, the sand resistance is at least 80%. Sand resistance is tested according to the following *in vivo* procedure, in which the SPF of a product before and after contact with sand for 30 minutes is compared. Specifically, 2 mg/cm² of product is applied by fingertip to the back of 10 healthy human subjects having a skin type of I to III according to the Fitzpatrick classification. Two grams of sand is then distributed on each treated area, which measures 30 cm², using a strainer to obtain a uniform grain size. A flat pouch having a weight of 300 g is then put on each treated area and left there for 30 minutes. The pouch is removed, and then the sand is carefully brushed off.

A solar simulator model 601 from Solar Light is used to irradiate the skin with six fibers corresponding to six different energies with a geometric progression of 1. A solar simulator model 601 from Solar Light is used to irradiate the skin with six fibers corresponding to six different energies with a geometric progression of 1.25 between each. A dosimeter model PMA from Solar Light is used to check the energy of each fiber before irradiation. SPF is measured without and with sand
contact. The ratio "SPF with sand contact to SPF without sand contact" is calculated as a percentage to obtain the remaining SPF activity.

The sunscreen product has water resistance of at least 80%. Water resistance is tested in a similar *in vivo* manner. SPF is measured without contact with a water bath, SPF is measured after contact with a water bath, and the ratio "SPF with bath to SPF without bath" is calculated as percentage to obtain the remaining SPF activity. The application of the product and the SPF measurement equipment are also the same as for the sand resistance test. Two baths lasting twenty minutes, with a 15 minute period in between, are used.

The sunscreen product also has an SPF of at least 2, in particular 2 to 60, more particularly about 10 to about 60.

In one embodiment, the sunscreen product comprises at least one silicon modified hydrolyzed protein. The silicon modified hydrolyzed protein may comprise a protein fragment selected from the group consisting of wheat proteins, collagen proteins, keratin proteins, fibroin proteins, and soy proteins. Silicon modified hydrolyzed wheat proteins are preferred. Silicon modified hydrolyzed proteins are commercially available, for example, from Seiwa Kasei Co., Ltd. as the PROMOIS SIG series.

In one embodiment of the invention, the sunscreen product comprises the silicon modified hydrolyzed wheat protein N-[2-hydroxy-3-[3-(dihydroxymethylsilyl)propyl]hydrolyzed wheat protein.

It is believed that silicon modified hydrolyzed proteins anchor the sunscreen product to the skin, providing it with excellent mechanical stability, i.e., ability to adhere. It is hypothesized that the protein portion of the compound connects to the skin, while the silicon-containing portion of the product links with the oil phase of the sunscreen product, which typically contains the actual sunscreens.

Another embodiment of the invention provides a composition comprising: a) from 0.1 to 10 weight percent of at least one silicon modified hydrolyzed protein; b) less than 3 weight percent of at least one film-forming agent; and c) from 1 to 30 weight percent of a sunscreen containing at least one UV-A absorber, at least one UV-B absorber, or at least one infrared protector.

A further embodiment of the invention provides a sunscreen product comprising: a) at least one silicon modified hydrolyzed protein and b) less than 3 weight percent of a film-forming agent; wherein said product has a rubbing resistance for at least 5 hours after a single application to mammalian skin and an SPF of at least 2.

Sunscreens useful in the present invention are compounds that absorb, reflect, or scatter radiation in the UV range. These include UV-A absorbers, UV-B absorbers and infrared protectors.

UV-B absorbers can be oil or water-soluble. As oil-soluble substances there can be mentioned for example:
- 3-Benzylidene campher, respectively 3-benzylidene norcampher and derivatives thereof, e.g. 3-(4-methylbenzylidene) campher;
- 4-Aminobenzoic acid derivatives, respectively 4-(dimethylamino)benzoic acid-2-ethylhexyl esters, 4-(dimethylamino)benzoic acid-2-octyl esters and 4-(dimethylamino)benzoic acid amylesters;
- Esters of cinnamonic acid, in particular 4-methoxycinnamonic acid-2-ethylhexylester, 4-methoxycinnamonicacid propylester, 4-methoxycinnamonic acid isoamyl ester, 2-cyano-3,3-phenylcinnamonic acid-2-ethylhexyl ester (octocrylene);
- Esters of salicylic acid, respectively salicylic acid-2-ethylhexylester, salicylic acid-4-isopropylbenzyl ester, salicylic acid homomenthyl ester;
- Derivatives of benzophenones, in particular 2-hydroxy-4-methoxybenzophenone, 2-hydroxy-4-methoxy-4'-methylbenzophenone, 2,2'-dihydroxy-4-methoxybenzophenone;
- Esters of benzalmalonic acid, in particular 4-methoxybenzmalonic acid di-2-ethylhexyl ester;
- Triazine derivatives, such as, for example, 2,4,6-trianilino-(p-carbo-2'-ethyl-1'-hexyloxy)-1,3,5-triazine and octyltriazone; or benzoic acid, 4,4'-[[6-[[[(1,1-Dimethylethyl)Amino]Carbonyl]Phenyl]Amino]-1,3,5-Triazine-2,4- diyl]Diimino]Bis-, Bis(2-Ethylhexyl) Ester (UVASORB HEB);
- Propane-1,3-diones, such as for example, 1-(4-tert.butylphenyl)-3-(4'-methoxyphenyl)propane-1,3-dione;
- Ketotricyclo(5.2.1.0)decane-derivatives.

Water-soluble UV-A and UV-B absorbers are for example:
- 2-Phenylbenzimidazol-5-sulfonic acid and its alkali-, alkaline earth-, ammonium-, alkylammonium-, alkanolammonium- and glucammonium salts;
- Sulfonic acid derivatives of benzophenones, in particular 2-hydroxy-4-methoxybenzophenone-5-sulfonic acid and its salts;
- Sulfonic acid derivatives of 3-benzylidene campher, e.g. 4-(2-oxo-3-bornylidene methyl)benzol-sulfonic acid and 2-methyl-5-(2-oxo-3-bornylidene)sulfonic acid and its salts.

Typical UV-A absorbers that can be used are derivatives of benzoylmethane, such as, for example, 1-(4'-tert.butylphenyl)-3-(4'-methoxyphenyl)propane-1,3-dione, 4-tert.-butyl-4'-methoxydibenzoylmethane (PARSOL 1789), 1-phenyl-3-(4'-isopropylphenyl)-propane-1,3-dione, derivatives of benzoic acid 2-(4 Diethylamino-2-Hydroxybenzoyl)-benzoic acid hexylester (UVINUL A+), or 1H-Benzimidazole-4,6-Disulfonic Acid, 2,2'-(1,4-Phenylene)Bis-, Disodium Salt (NEO HELOPAN AP ).

Mixtures of UV-A and UV-B absorbers can be used too.

Of particular interest are the so-called broadband filters. One type of such filters are the water-soluble filters, more specifically the benzotriazoles, in particular the benzotriazole derivate known as 2,2'-methylene-bis- (6-(2H- benzotriazole-2-yl)-4-(1,1,3,3-tetramethylbutyl)-phenol) [INCI: Bisoctyltriazol], which is commercially available under the tradename TINOSORB M from CIBA Chemicals. Another useful benzotriazole derivate is 2-(2H-benzotriazole-2-yl)-4-methyl-6-[2-methyl-3-[1,3,3,3- tetramethyl-1-[(trimethylsilyl)oxy]disiloxanyl]propyl]-phenol (CAS-No.: 155633-54-8) also indicated by the INCI name drometrizole trisiloxane and is available from Chimex under the tradename MEXORYL XL. These benzotriazole derivatives can be conveniently incorporated in the water phase at a pH above 4.5.

Other useful water-soluble UV absorbers are the sulfonated UV filters such as 3,3'-(1,4- phenylenedimethylene) bis (7,7-dimethyl-2-oxo-bicyclo-[2.2.1]hept-1-yl methanesulfonic acid, and its sodium, potassium or its triethanolammonium salt, and the sulfonic acid itself, indicated by the INCI name terephthalidene dicamphor sulfonic acid (CAS No. 90457- 82-2), which is available, for example, under the trade name MEXORYL SX from Chimex.

Oil-soluble broadband filters are the asymmetrically substituted triazine derivatives. Of particular interest is 2,4- Bis-{[4-(2-ethyl-hexyloxy)-2-hydroxy]-phenyl}-6-(4-methoxyphenyl)-1,3,5-triazine (INCI: anisotriazine), that is commercially available under the tradename TINOSORB S from CIBA Chemicals.

A further advantageous water-soluble UV filter is 3,3'-(1,4-phenylenedimethylene) bis (7,7-dimethyl-2-oxo-bicyclo-[2.2. 1]hept-1-ylmethanesulfonic acid, as well its sodium, potassium or its triethanolammonium salt, with the INCI name terephthalidene dicamphor sulfonic acid (CAS No. 90457-82-2), which is available, for example, under the trade name MEXORLY SX from Chimex.

Examples of inorganic pigment filters includeinsoluble pigments, namely finely dispersed metal oxides or metal salts. Examples of appropriate metal oxides in particular are zinc oxide and titanium dioxide as well as oxides of iron, zirconium, silicon, manganese, aluminium and cerium as well as mixtures thereof. Salts that can be used comprise silicates (talcum), barium sulfate or zinc stearate. The particle size of these pigments is sufficiently small, e.g. less than 100 nm, in particular between 5 and 50 nm and more in particular between 15 and 30 nm. The particles can be spherical but can have other shapes too such as ellipsoidal or similar shapes. The surface of the pigments may have been treated, e.g. hydrophilized or made hydrophobic. Typical examples are coated titanium dioxide, e.g. Titanium dioxide T 805 (available from Degussa) or EUSOLEX T 2000 (Merck). Silicones can be used as hydrophobic coating agents, in particular trialkoxyoctyl silanes or simethicones. So-called micro- or nanopigments are particularly attractive for use in sunscreen products.

Examples of film-forming agents include, but are not limited to, PVP, PVP/Eicosene copolymer, tricontanyl PVP, PVP/hexadecene copolymer, hydrogenated polyisobutene, adipic acid/diethylene glycol/glycerin crosspolymer, octadecene/MA copolymer, synthetic wax, diglycol/CHDM/Isophthalates/SIP copolymer, and mixtures thereof.

Advantageously, the present invention allows for the use of a lower level of film-forming agents than are typically found in known sunscreen products. According to the invention, less than 3 weight percent of film-forming agent may be present in the products and compositions of the invention. More particularly, from 0.01 to 3 weight percent of film-forming polymer may be used. These reduced amounts are advantageous in that they result in products and compositions that are more stable and have better aesthetics such as lighter feel, increased creaminess, and reduced stickiness.

The products and compositions of the invention may optionally contain other cosmetically-acceptable, active agents in addition to the sunscreen(s). These include compounds (e.g., synthetic compounds or compounds isolated from natural sources) having cosmetic or therapeutic effects on the skin, hair, or nails, including, but not limiting to, lightening agents, darkening agents such as self-tanning agents, propigmenting agents, anti-acne agents, shine control agents, anti-microbial agents, anti-inflammatory agents, anti-mycotic agents, anti-parasite agents, external analgesics, antioxidants, keratolytic agents, detergents/surfactants, moisturizers, nutrients, vitamins, energy enhancers, anti-perspiration agents, astringents, deodorants, hair removers, firming agents, anti-wrinkle agents, anti-sagging agents, anti-callous agents, and agents for hair, nail, and/or skin conditioning.

In one embodiment, the cosmetically-acceptable, active agent is selected from, but not limited to, the group consisting of hydroxy acids, benzoyl peroxide, D-panthenol, carotenoids, free radical scavengers, spin traps, retinoids such as retinol, retinaldehyde, and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q 10, amino acids such a proline, vitamins, lactobionic acid, acetyl-coenzyme A, niacin, riboflavin, thiamin, ribose, electron transporters such as NADH and FADH2, and other botanical extracts such as aloe vera, and derivatives, soya extracts, and mixtures thereof.

Such cosmetically-active agents are typically present in the products and compositions of the invention in an amount of from 0.001% to 20% by weight of the composition, e.g., from 0.005% to 10% such as from 0.01% to 5%.

Examples of vitamins include, but are not limited to, vitamin A, vitamin Bs such as vitamin B3, vitamin B5, and vitamin B 12, vitamin C, vitamin K, and vitamin E and derivatives thereof.

Examples of hydroxy acids include, but are not limited, to glycolic acid, lactic acid, malic acid, salicylic acid, citric acid, and tartaric acid. See, e.g., European Patent Application No. 273,202.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Suitable oil-soluble antioxidants include, but are not limited to, butylated hydroxytoluene, retinoids (e.g., retinal, retinaldehyde, and retinyl palmitate), tocopherols (e.g., tocopherol acetate), tocotrienols, and ubiquinone. Suitable natural extracts containing antioxidants include, but are not limited to, extracts containing flavonoids and isoflavonoids and their derivatives (e.g., genistein and diadzein), extracts containing resveratrol and the like. Examples of such natural extracts include grape seed, green tea, pine bark, and propolis.

Various other materials may also be present in the products and compositions, including humectants, proteins and polypeptides, chelating agents (e.g., EDTA), and preservatives (e.g., parabens), and pH adjusting agents. In addition, the products and compositions may contain conventional cosmetic adjuvants, such as dyes, opacifiers (e.g., titanium dioxide), pigments, and fragrances.

The products and compositions of the invention are preferably in the form of oil in water (O/W) emulsions. The O/W emulsions contain an oil phase that may comprise suitable oils which are skin-compatible components or mixtures that are non water-miscible. Preferably, the oils are liquid at ambient temperature, in particular are liquid at 25 °C. They can contain certain amounts of solid lipid components (e.g. fats or waxes) as long as the complete oily mixture is liquid at ambient temperature or at the temperature mentioned above.

The water phase in the O/W emulsions of this invention may be pure water but usually contains one or more hydrophilic components. The latter can be lower alkanols, polyols, water-soluble active ingredients, preservatives and moisturizers, chelating agents, etc.

The products and compositions may be optionally prepared using a mineral water, for example mineral water that has been naturally mineralized such as EVIAN Mineral Water (Evian, France). In one embodiment, the mineral water has a mineralization of at least 200 mg/L (e.g., from 300 mg/L to 1000 mg/L). In one embodiment, the mineral water contains at least 10 mg/L of calcium and/or at least 5 mg/L of magnesium.

The products and compositions of the present invention may be prepared using methodology that is well known by an artisan of ordinary skill. The products and compositions may be put into finished packaged form. In one embodiment, the package is a container such as a plastic, metal or glass tube or jar containing the product or composition. The product or composition may further comprise additional packaging such as a plastic or cardboard box for storing such container. In one embodiment, the product or composition contains instructions directing the user to apply the product or composition to the skin to provide protection from ultraviolet light (e.g., UV-A and/or UV-B light from the sun). Such instructions may be printed on the container, label insert, or on any additional packaging.

The products and compositions may be promoted by advertising or marketing. Examples of promoting include, but are not limited to, written, visual, or-verbal statements made on the product or composition or in stores, magazines, newspaper, radio, television, internet, and the like. Examples of such statements include, but are not limited to, "sunscreen," "sunblock," "sunprotection," "ultraviolet protection," "ultraviolet block," "all day performance," long lasting performance," "10 hour performance," and the like.

The products and compositions are topically applied by means of directly laying on or spreading on outer skin, nails, or hair, e.g., by use of the hands or an applicator such as a wipe, roller, or spray.

### Example 1

The sand resistance and water resistance of an SPF 30 sunscreen product according to the invention were measured using the test methods described above. The sunscreen product contained 2 weight percent PROMOIS WG-SIG (N-[2-hydroxy-3-[3-(dihydroxymethylsilyl)propyl]hydrolyzed wheat protein), commercially available from Seiwa Kasei Co., Ltd.

A comparative 30 SPF sunscreen product containing no silicon modified hydrolyzed protein was also tested for sand resistance and water resistance.

The comparative product had a sand resistance of 60% and a water resistance of 80%. In contrast, the sunscreen product of the invention had a sand resistance of 82% and a water resistance of 84%.

### Examples 2 and 3

An SPF 30 sunscreen product and an SPF 10 sunscreen product according to the invention were made. The SPF (UV-B protection) and PFA (UV-A protection) remaining after 10 hours were measured in clinical tests as follows.

SPF after 10 hours was evaluated based on the Colipa Method using 2 mg/cm² of product, but instead of applying the whole energy (Xenon lamp ) at one time, the energy was divided into four doses (one dose every two hours during 10 hours) to more closely simulate actual sun exposure conditions. The Erythema reading was done 24H after the last exposure. This SPF was compared to the SPF obtained with the Colipa method, and the ratio:(SPF 10 hours/SPF)*100 gave the % of SPF remanence.

PFA after 10 Hours was evaluated based on IPD method (immediate pigmentation darkening). As with the SPF determination, the energy was applied in four doses (one dose every two hours during 10 hours) to more closely simulate actual sun exposure conditions. The pigmentation reading was done immediately after the last exposure. This PFA was compared to the classical PFA, and the ratio:(PFA 10 hours/PFA)*100 gave the % of PFA remanence.

The results were as follows. Sunscreen products according to the invention had a global remanence for at least 10 hours.

| | SPF remanence after 10 hours | PFA remanence after 10 hours |
|---|---|---|
| Example 2: | 34.5 (94.4%) | 11.8 (84.4%) |
| Example 3: | 15.2 (92.2%) | 4.9 (100%) |

The composition of Example 2 was as follows:

| INGREDIENT | % |
|---|---|
| Aqua | 57.02 |
| Acrylates/Steareth-20 Methacrylate Copolymer | 1.70 |
| aqua | |
| Sodium Lauryl Sulfate | |
| Xanthan Gum | 0.15 |
| Butylene Glycol | 3.00 |
| Phenoxyethanol | 0.80 |
| Caprilyl Glycol | 0.50 |
| Glycerin | 2.00 |
| Disodium Edta | 0.10 |
| Hydrolyzed Wheat Protein PG-Propyl Methylsilanediol | 2.00 |
| Butylene Glycol | |
| Aqua | |
| Iodopropynyl Butylcarbamate | 0.08 |
| PEG-4 Laurete | |
| PEG-Dilaurate | |
| PEG-4 | |
| PEG-100 Stearate | 3.00 |
| Glyceryl Stearate | |
| Cetearyl Alcohol | 0.25 |
| PVP-Eicosene Copolymer | 1.00 |
| C12-15 Alkyl Benzoate | 7.00 |
| Ethylhexyl Methoxycinnamate | 6.50 |
| 4-Methylbenzylidene Camphor | 4.00 |
| Bis-Ethylhexyloxyphenol Methoxyphenyl Triazine | 2.00 |
| Dimethicone | 3.00 |
| Tocopheryl Acetate | 0.50 |
| Methylene Bis-Benzotriazolyl Tetramethybutylphenol | 2.00 |
| Aqua | |
| Decyl Glucoside | |
| Xanthan Gum | |
| Aqua | 2.00 |
| Citic Acid | 0.02 |
| Sodium Hydroxide | 0.08 |
| Aqua | 1.00 |
| Perfum | 0.3000 |
| 0 | 0.00 |
| | **100.00** |
| | |

The composition of Example 3 was as follows:

| INGREDIENT | % |
|---|---|
| Aqua | 67.86 |
| Acrylates/Steareth-20 Methacrylate Copolymer | 1.75 |
| Aqua | |
| Sodium Lauryl Sulfate | |
| Xanthan Gum | 0.15 |
| Butylene Glycol | 3.00 |
| Phenoxyethanol | 0.80 |
| Caprilyl Glycol | 0.50 |
| Glycerin | 2.00 |
| Metylparaben | 0.30 |
| Disodium Edta | 0.10 |
| Hydrolyzed Wheat Protein PG-Propyl Methylsilanediol | 2.00 |
| Butylene Glycol | |
| Aqua | |
| PEG-100 Stearate | 2.50 |
| Glyceryl Stearate | |
| Cetearyl Alcohol | 0.15 |
| PVP-Eicosene Copolymer | 1.00 |
| C12-15 Alkyl Benzoate | 7.00 |
| Ethylhexyl Methoxycinnamate | 1.00 |
| 4-Methylbenzylidene Camphor | 2.00 |
| Bis-Ethylhexyloxyphenol Methoxyphenyl Triazine | 1.00 |
| Dimethicone | 3.00 |
| Tocopheryl Acetate | 0.50 |
| Methylene Bis-Benzotriazolyl Tetramethybutylphenol | 1.00 |
| Aqua | |
| Decyl Glucoside | |
| Xanthan Gum | |
| Aqua | 1.00 |
| Citric Acid | 0.01 |
| Sodium Hydroxide | 0.08 |
| Aqua | 1.00 |
| Perfum | 0.30 |
| | |
| | **100.00** |
| | |

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A sunscreen product having rubbing resistance for at least 5 hours after a single application to the mammalian skin or hair and an SPF of at least 2 and comprising at least one silicone modified hydrolyzed protein, at least one film-forming agent, and a sunscreen which comprises at least one UV-A-absorber, UV-B-absorber, infrared protector, or a combination thereof.

2. The sunscreen product according to claim 1 wherein the silicone modified hydrolyzed protein is present in the range from 0.1 to 10 wt.-%, the film-forming agent is present in an amount of less than 3 wt.-%, and the sunscreen is present in the range from 1 to 30 wt.-%.

3. The sunscreen product of claim 1 or 2, wherein the silicon modified hydrolyzed protein comprises a protein fragment selected from the group consisting of wheat proteins, collagen proteins, keratin proteins, fibroin proteins, and soy proteins.

4. The sunscreen product of claim 1 comprising a silicone modified hydrolyzed wheat protein.

5. The sunscreen product of claim 4, wherein the silicone modified hydrolyzed wheat protein is N-[2-hydroxy-3-[3-(dihydroxymethylsilyl)propyl]hydrolyzed wheat protein.

6. The sunscreen product of claim 1 having a sand resistance of at least 65%.

7. The sunscreen product of claim 6 having a water resistance of at least 50%.

8. The sunscreen product of claim 1 having a global remanence of at least 8 hours.

9. The sunscreen product according to claim 2 comprising 0.1 to 30 weight percent lipophilic sunscreens; 0.1 to 10 weight percent hydrophilic sunscreens; and 1 to 15 weight percent inorganic pigment filters.

## Patentansprüche

1. Sonnenschutzprodukt mit einer Abriebbeständigkeit von mindestens 5 Stunden nach einem einmaligen Auftragen auf die Haut oder das Haar eines Säugetiers und einem SPF von mindestens 2, das mindestens umfasst: ein mit Silikon modifiziertes hydrolysiertes Protein, mindestens einen Filmbildner und ein Sonnenschutzmittel, das mindestens ein UVA-Absorptionsmittel, ein UVB-Absorptionsmittel, ein Infrarotschutzmittel oder eine Kombination derselben umfasst.

2. Sonnenschutzprodukt nach Anspruch 1, **dadurch gekennzeichnet, dass** das mit Silikon modifizierte hydrolysierte Protein im Bereich von 0,1 bis 10 Gew.-% vorliegt, der Filmbildner in einer Menge von weniger als 3 Gew.-% vorliegt und das Sonnenschutzmittel im Bereich von 1 bis 30 Gew.-% vorliegt.

3. Sonnenschutzprodukt nach Anspruch 1 oder 2, **dadurch gekennzeichnet, dass** das mit Silikon modifizierte hydrolysierte Protein ein Proteinfragment umfasst, das ausgewählt ist aus der Gruppe bestehend aus Weizenproteinen, Kollagenproteinen, Keratinproteinen, Fibroinproteinen und Sojaproteinen.

4. Sonnenschutzprodukt nach Anspruch 1, das ein mit Silikon modifiziertes hydrolysiertes Weizenprotein umfasst.

5. Sonnenschutzprodukt nach Anspruch 4, **dadurch gekennzeichnet, dass** das mit Silikon modifizierte hydrolysierte Weizenprotein mit N-[2-Hydroxy-3-[3-(dihydroxymethylsilyl)propyl] hydrolysiertes Weizenprotein ist.

6. Sonnenschutzprodukt nach Anspruch 1 mit einer Sandfestigkeit von mindestens 65 %.

7. Sonnenschutzprodukt nach Anspruch 6 mit einer Wasserfestigkeit von mindestens 50%.

8. Sonnenschutzprodukt nach Anspruch 1 mit einer Gesamtverweildauer von mindestens 8 Stunden.

9. Sonnenschutzprodukt nach Anspruch 2, das 0,1 bis 30 Gewichtsprozent lipophile Sonnenschutzmittel, 0,1 bis 10 Gewichtsprozent hydrophile Sonnenschutzmittel und 1 bis 15 Gewichtsprozent anorganische Pigmentfilter umfasst.

## Revendications

1. Produit solaire présentant une résistance au frottement d'au moins 5 heures après une seule application sur la peau ou les cheveux de mammifères et un SPF d'au moins 2 et comprenant au moins une protéine hydrolysée modifiée par de la silicone, au moins un agent filmogène et un écran solaire qui comprend au moins un absorbeur d'UV-A, un absorbeur d'UV-B, un protecteur infrarouge ou une combinaison de ceux-ci.

2. Produit solaire selon la revendication 1, dans lequel la protéine hydrolysée modifiée par de la silicone est présente dans la plage de 0,1 à 10 % en poids, l'agent filmogène est présent dans une quantité inférieure à 3 % en poids et l'écran solaire est présent dans la plage de 1 à 30 % en poids.

3. Produit solaire selon la revendication 1 ou 2, dans lequel la protéine hydrolysée modifiée par de la silicone comprend un fragment de protéine choisi dans le groupe constitué par les protéines de blé, les protéines de collagène, les protéines de kératine, les protéines de fibroïne et les protéines de soja.

4. Produit solaire selon la revendication 1, comprenant une protéine de blé hydrolysée modifiée par de la silicone.

5. Produit solaire selon la revendication 4, dans lequel la protéine de blé hydrolysée modifiée par de la silicone est la protéine de blé hydrolysée modifiée par du N-[2-hydroxy-3-[3-(dihydroxyméthylsilyl)propyle].

6. Produit solaire selon la revendication 1, ayant une résistance au sable d'au moins 65 %.

7. Produit solaire selon la revendication 6, ayant une résistance à l'eau d'au moins 50 %.

8. Produit solaire selon la revendication 1, ayant une rémanence globale d'au moins 8 heures.

9. Produit solaire selon la revendication 2, comprenant de 0,1 à 30 pour cent en poids d'écrans solaires lipophiles ; 0,1 à 10 pour cent en poids d'écrans solaires hydrophiles ; et 1 à 15 pour cent en poids de filtres pigmentaires minéraux.
